# EUROPEAN PATENT APPLICATION

(11) **EP 4 776 804 A1**
(43) Date of publication of application: **15.07.2026**
(21) Application number: 24862870.3
(22) Date of filing: 05.09.2024
(51) Int. Cl.: H10K 50/858, C07D 413/10, C07D 413/14, C07D 417/14, C07D 498/04, H10K 50/15, H10K 50/16, H10K 50/81, H10K 50/82, H10K 85/60

(54) **BENZOAZOLE COMPOUND AND ORGANIC ELECTROLUMINESCENT ELEMENT USING SAID COMPOUND**

(30) Priority: 08.09.2023 JP 2023145921
(71) Applicant: Hodogaya Chemical Co., Ltd., Tokyo, 105-0021 (JP)
(72) Inventor: KASE, Kouki, Tokyo 105-0021 (JP); YANG, Byung-Sun, Tokyo 105-0021 (JP); CHA, Hyun-Wook, Tokyo 105-0021 (JP); KIM, Hee-Jae, Tokyo 105-0021 (JP); HIRAYAMA, Yuta, Tokyo 105-0021 (JP); HAYASHI, Shuichi, Tokyo 105-0021 (JP)
(74) Representative: Meissner Bolte Partnerschaft mbB
(86) International application number: PCT/JP2024/031873
(87) International publication number: WO 2025/053219

(57) **Abstract**

Provided is an organic electroluminescent (EL) device in which, in order to improve the light extraction efficiency of the organic EL device, a compound having a high refractive index at a wavelength of 450 nm to 750 nm and a low extinction coefficient is used in a capping layer. The organic EL device has an anode, a hole transport layer, a light-emitting layer, an electron transport layer, a cathode, and a capping layer in that order, wherein the capping layer contains a benzazole compound represented by the following general formula (1): 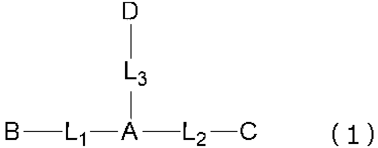 wherein A represents a benzoxazolyl ring or the like, B and C represent a phenyl group, naphthyl group, phenanthrolinyl group, oxazolopyridyl group, or the like, D represents either a hydrogen atom or an aryl group or the like, and L₁ to L₃ represent either a single bond or a divalent aryl group or the like.

## Description

### TECHNICAL FIELD

The present invention relates to benzazole compounds suitable for self-luminous electronic devices for various display devices, particularly organic electroluminescent devices (hereinafter abbreviated to as "organic EL devices"), as well as to organic EL devices, electronic apparatus, and electronic devices using these compounds.

### BACKGROUND ART

Organic EL devices have been actively studied due to their higher brightness, superior visibility, and their ability to display clearer images compared with liquid crystal devices. In 1987, C.W. Tang et al. of Eastman Kodak Company developed a laminated structure device in which different materials were assigned specific roles, enabling practical application of an organic EL device using organic materials. To date, many improvements have been made for the practical application of organic EL devices. By further subdividing the roles of each layer in a laminated structure and sequentially providing, on a substrate, an anode, a hole injection layer, a hole transport layer, a light-emitting layer, an electron transport layer, an electron injection layer, and a cathode, a bottom-emission-type light-emitting device that emits light from the bottom has been designed, thereby achieving high efficiency and durability (see Non-Patent Document 1).

Recently, top-emission type light-emitting devices, in which a metal having a high work function is used for an anode and light is emitted from the top, have come into use. In bottom-emission type light-emitting devices, light is extracted from the bottom that has pixel circuits, and the area of the light-emitting portion is therefore limited. In contrast, in a top-emission type light-emitting device, light is extracted from the top, whereby the pixel circuit does not block the light and the light-emitting area can be made larger. On the other hand, in top-emission type light-emitting devices, there has been a limitation in that, when light emitted from the light-emitting layer is incident on another layer at a certain angle or more, total internal reflection occurs at the interface between the light-emitting layer and the other layer, with the result that only a portion of the emitted light can be utilized. Therefore, in order to improve the light extraction efficiency, light-emitting devices have been proposed in which a capping layer having a high refractive index is provided outside a semi-transparent electrode (cathode) having a low refractive index such as LiF/Al/Ag, Ca/Mg, or LiF/MgAg (see Non-Patent Document 2 and Non-Patent Document 3).

### CITATION LIST

### PATENT LITERATURE

[PTL 1] WO 2014/009310
[PTL 2] WO 2018/008718
[PTL 3] WO 2013/179536

### NON-PATENT LITERATURE

[NPL 1] Proceedings of the 9th Tutorial Meeting of the Japan Society of Applied Physics, 2001, pp. 55-61
[NPL 2] Appl. Phys. Lett., 2001, Vol. 78, No. 4, pp. 544-546
[NPL 3] Appl. Phys. Lett., 2003, Vol. 82, No. 3, pp. 466-468
[NPL 4] Tetrahedron, 2002, Vol. 58, pp. 9633-9695
[NPL 5] J. Org. Chem., 2006, Vol. 71, pp. 1802-1808
[NPL 6] Appl. Phys. Lett., 2011, Vol. 98, No. 8, 083302

### SUMMARY OF INVENTION

### TECHNICAL PROBLEM

Conventionally, a high-definition metal mask has been used for forming the capping layer. However, when used under high-temperature conditions, the heat causes distortion of the metal mask, resulting in a decrease in alignment accuracy. Therefore, ZnSe (melting point: 1100°C or more), which is used for the capping layer in Non-Patent Document 3, cannot be vapor-deposited at an accurate position when a high-definition metal mask is used and this inaccuracy may adversely affect the light-emitting device. Furthermore, inorganic materials are not suitable as constituent materials of the capping layer because film formation by the sputtering method also adversely affects the light-emitting device.

Non-Patent Document 2 proposes the use of tris(8-hydroxyquinoline) aluminum (Alq₃) as a constituent material of the capping layer for adjusting the refractive index. However, Alq₃ is a material commonly used as a green-light-emitting material or as an electron transport material and exhibits weak absorption in the vicinity of 450 nm, which is close to the emission wavelength of blue-light-emitting materials. Therefore, a problem occurs in that color purity and light extraction efficiency are reduced when Alq₃ is used in a blue-light-emitting device.

As described above, in order to improve the device characteristics and the light extraction efficiency of organic EL devices, there is a need for materials that can be used for a capping layer that have a high refractive index and low extinction coefficient, as well as excellent thin-film stability, film-forming properties, and durability.

An object of the present invention is to provide a compound having a high refractive index in the wavelength range of 450 nm to 750 nm and a low extinction coefficient, and further, that is suitable as a material for a capping layer of an organic EL device. Another object of the present invention is to provide an organic EL device having improved light extraction efficiency compared to conventional devices.

### SOLUTION TO PROBLEM

In order to achieve the above objects, the present inventors diligently optimized the molecular design by focusing on the fact that benzazole compounds exhibit excellent thin-film stability and durability. As a result, the present invention was achieved by developing a material having a high refractive index and a low extinction coefficient in the wavelength range of 450 nm to 750 nm. Specifically, the present invention includes the following configuration:
1) An organic electroluminescent device, comprising an anode, a hole transport layer, a light-emitting layer, an electron transport layer, a cathode, and a capping layer in that order, wherein the capping layer comprises a benzazole compound represented by the following general formula (1): 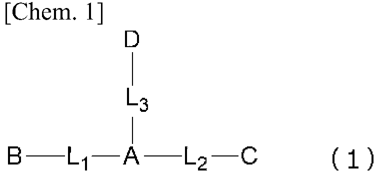
   wherein A represents a benzoxazolyl ring or benzothiazolyl ring,
   B and C each independently represent a substituted or unsubstituted phenyl group, naphthyl group, quinolyl group, quinoxalinyl group, quinazolinyl group, phenanthrolinyl group, benzoxazolyl group, benzothiazolyl group, benzofuryl group, benzothienyl group, or oxazolopyridyl group,
   D represents either a hydrogen atom or a substituted or unsubstituted aryl group or heteroaryl group, and
   L₁ to L₃ each independently represent either a single bond or an unsubstituted divalent aryl group or divalent heteroaryl group.
2) The organic electroluminescent device according to 1), wherein L₂, L₃, and L₁ in general formula (1) are bonded to the 2-position, 4-position, and 6-position, respectively, of the benzoxazolyl ring or benzothiazolyl ring represented by A.
3) The organic electroluminescent device according to 1) or 2), wherein L₁ to L₃ in general formula (1) are each independently either a single bond or an unsubstituted phenylene group, biphenylylene group, or naphthylene group.
4) The organic electroluminescent device according to any one of 1) to 3), wherein the aryl group or heteroaryl group represented by D in general formula (1) is selected from a group consisting of a phenyl group, biphenylyl group, naphthyl group, furyl group, quinolyl group, benzoxazolyl group, benzothiazolyl group, oxazolopyridyl group, quinoxalinyl group, quinazolinyl group, and phenanthrolinyl group.
5) The organic electroluminescent device according to any one of 1) to 4), wherein D in general formula (1) is a hydrogen atom, a cyanophenyl group, or an unsubstituted phenyl group, biphenylyl group, naphthyl group, furyl group, quinolyl group, benzoxazolyl group, benzothiazolyl group, oxazolopyridyl group, quinoxalinyl group, quinazolinyl group, or phenanthrolinyl group.
6) The organic electroluminescent device according to any one of 1) to 5), wherein at least one of B and C in general formula (1) is a group selected from a group consisting of an unsubstituted phenyl group, naphthyl group, quinolyl group, quinazolinyl group, phenanthrolinyl group, benzoxazolyl group, benzothiazolyl group, and oxazolopyridyl group.
7) The organic electroluminescent device according to any one of 1) to 6), wherein when the capping layer is formed to a thickness of 30 nm to 120 nm, the refractive index of the formed film is 1.70 or more in a wavelength range of 450 nm to 750 nm.
8) The organic electroluminescent device according to any one of 1) to 7), wherein the capping layer is a laminated layer or a mixed layer comprising two or more kinds of compounds including a benzazole compound represented by general formula (1).
9) A benzazole compound represented by the following general formula (2): 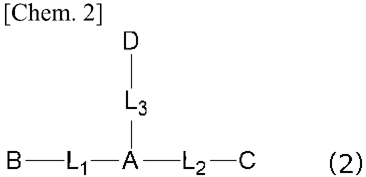
   wherein A represents a benzoxazolyl ring or benzothiazolyl ring,
   B and C each independently represent a substituted or unsubstituted phenyl group, naphthyl group, quinolyl group, quinoxalinyl group, quinazolinyl group, phenanthrolinyl group, benzoxazolyl group, benzothiazolyl group, benzofuryl group, benzothienyl group, or oxazolopyridyl group,
   D represents either a hydrogen atom or a substituted or unsubstituted phenyl group, biphenylyl group, naphthyl group, furyl group, quinolyl group, quinoxalinyl group, quinazolinyl group, phenanthrolinyl group, benzoxazolyl group, benzothiazolyl group, benzofuryl group, benzothienyl group, or oxazolopyridyl group,
   L₁ to L₃ each independently represent either a single bond or an unsubstituted phenylene group, biphenylylene group, or naphthylene group, and
   L₂, L₃, and L₁ are bonded to the 2-position, 4-position, and 6-position, respectively, of the benzoxazolyl ring or benzothiazolyl ring represented by A,
   wherein the compound satisfies either Requirement 1 or 2 below:
      Requirement 1: At least one of B, C, and D contains a structure selected from a group consisting of quinazoline, phenanthroline, and oxazolopyridine, provided that:
         if one or two of B, C, and D are substituted or unsubstituted phenanthrolinyl groups and none of the one or two of B, C, and D that are not the phenanthrolinyl groups is a group selected from a substituted or unsubstituted quinazolinyl group and oxazolopyridyl group, then at least one of L₁, L₂, and L₃ bonded to the phenanthrolinyl groups is an unsubstituted phenylene group, biphenylylene group, or naphthylene group; and
         if B, C, and D are all substituted or unsubstituted phenanthrolinyl groups, at least one selected from L₁, L₂, and L₃ bonded to the phenanthrolinyl groups is an unsubstituted phenylene group, biphenylylene group, or naphthylene group; and
      Requirement 2: None of B, C, and D contains any structure selected from a group consisting of quinazoline, phenanthroline, and oxazolopyridine, L₃ is a single bond, and D is a hydrogen atom.
10) The benzazole compound according to 9), wherein at least one of B and C in general formula (2) is a group selected from a group consisting of a substituted or unsubstituted phenyl group, naphthyl group, quinolyl group, quinazolinyl group, phenanthrolinyl group, benzoxazolyl group, benzothiazolyl group, benzofuryl group, and oxazolopyridyl group.
11) An electronic apparatus or an electronic device, comprising a pair of electrodes and at least one organic layer, wherein the organic layer comprises a benzazole compound according to 9) or 10).

### ADVANTAGEOUS EFFECTS OF INVENTION

The benzazole compound according to the present invention has a high refractive index in the wavelength range of 450 nm to 750 nm, a low extinction coefficient, can be vapor-deposited, is stable in a thin-film state, and has high heat resistance. Therefore, by providing a capping layer containing the compound on the outside of the electrode of the organic EL device, the light extraction efficiency can be improved compared with conventional devices.

### BRIEF DESCRIPTION OF DRAWINGS

[Fig. 1] Fig. 1 is a diagram showing specific examples of benzazole compounds represented by general formula (1).
[Fig. 2] Fig. 2 is a diagram showing specific examples of benzazole compounds represented by general formula (1).
[Fig. 3] Fig. 3 is a diagram showing specific examples of benzazole compounds represented by general formula (1).
[Fig. 4] Fig. 4 is a diagram showing specific examples of benzazole compounds represented by general formula (1).
[Fig. 5] Fig. 5 is a diagram showing specific examples of benzazole compounds represented by general formula (1).
[Fig. 6] Fig. 6 is a diagram showing specific examples of benzazole compounds represented by general formula (1).
[Fig. 7] Fig. 7 is a diagram showing specific examples of benzazole compounds represented by general formula (1).
[Fig. 8] Fig. 8 is a diagram showing specific examples of benzazole compounds represented by general formula (1).
[Fig. 9] Fig. 9 is a diagram showing specific examples of benzazole compounds represented by general formula (1).
[Fig. 10] Fig. 10 is a diagram showing an example of the configuration of an organic EL device of the present invention.

### DESCRIPTION OF EMBODIMENTS

### Organic EL Device

The organic EL device of the present invention includes at least an anode, a hole transport layer, a light-emitting layer, an electron transport layer, a cathode, and a capping layer in that order.

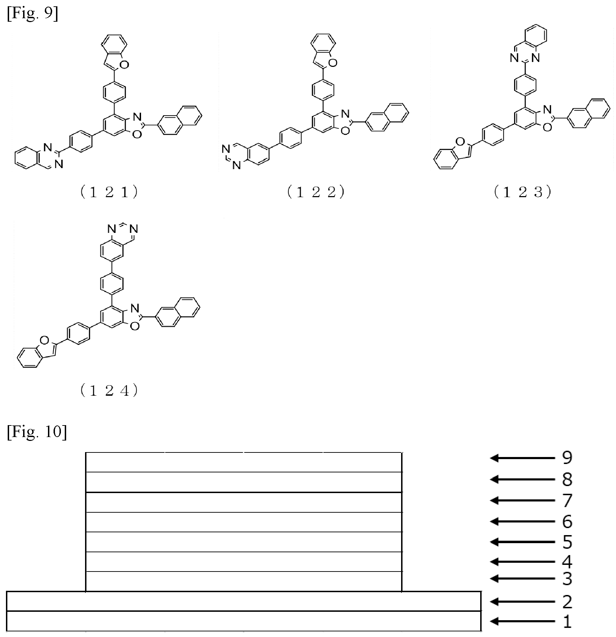

As an example of the structure of the organic EL device, in the case of a top-emission type light-emitting device, a structure can be cited in which anode 2, hole transport layer 4, light-emitting layer 5, electron transport layer 6, cathode 8, and capping layer 9 are sequentially laminated on glass substrate 1, as shown in Fig. 10. Also included are devices having hole injection layer 3 between anode 2 and hole transport layer 4, devices having an electron-blocking layer (not shown) between hole transport layer 4 and light-emitting layer 5, devices having a hole-blocking layer (not shown) between light-emitting layer 5 and electron transport layer 6, and devices having electron injection layer 7 between electron transport layer 6 and cathode 8. In other words, the organic EL device of the present invention does not exclude embodiments in which other layers are present between each layer, provided that the device includes at least an anode, a hole transport layer, a light-emitting layer, an electron transport layer, a cathode, and a capping layer in that order. In these multilayer structures, a single organic layer can perform multiple functions. For example, a layer may serve as both a hole injection layer and a hole transport layer, a hole transport layer and an electron-blocking layer, a hole-blocking layer and an electron transport layer, and/or an electron transport layer and an electron injection layer. Furthermore, organic layers having the same function may be laminated in two or more layers. Specifically, examples include configurations in which two hole transport layers, two light-emitting layers, two electron transport layers, and/or two capping layers are laminated. Each of the layers constituting the organic EL device is described below.

### Capping Layer

In the organic EL device of the present invention, the capping layer contains at least a benzazole compound represented by the following general formula (1).

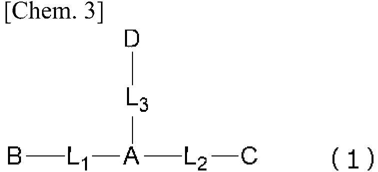

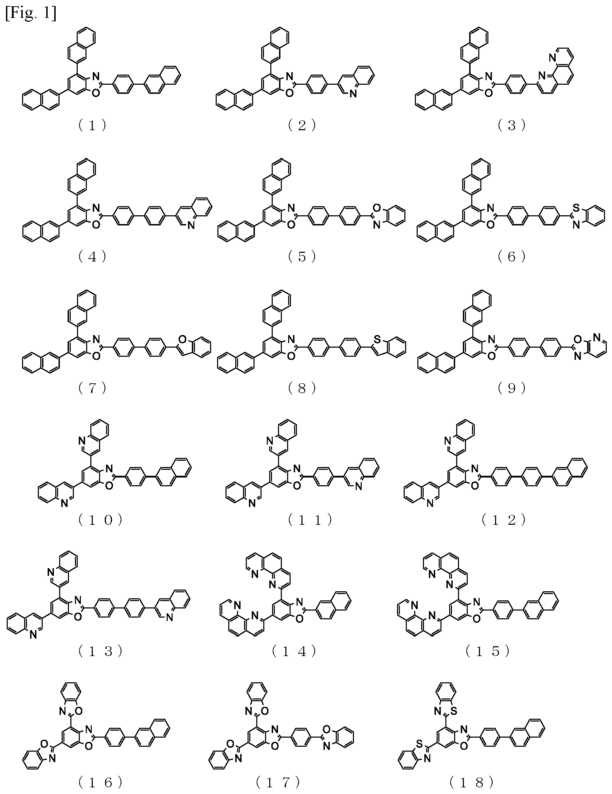

In general formula (1), A represents a benzoxazolyl ring or benzothiazolyl ring and is bonded to L₁, L₂, and L₃ on the carbon atoms that constitute the ring. For example, as in compound (1) shown in Fig. 1, L₂, L₃, and L₁ are preferably bonded to the 2-position, 4-position, and 6-position, respectively, of the benzoxazolyl ring or benzothiazolyl ring represented by A. Moreover, A is preferably a benzoxazolyl ring.

B and C each independently represent a substituted or unsubstituted group selected from the following groups: a phenyl group, naphthyl group, quinolyl group, quinoxalinyl group, quinazolinyl group, phenanthrolinyl group, benzoxazolyl group, benzothiazolyl group, benzofuryl group, benzothienyl group, or oxazolopyridyl group. The oxazolopyridyl group may be an oxazolo[4,5-b]pyridyl group or an oxazolo[5,4-b]pyridyl group.

Specifically, examples of "substituents" that each of the above substituted or unsubstituted groups represented by B and C may have include the following atoms or groups:
Deuterium atoms; halogen atoms such as fluorine, chlorine, bromine, and iodine atoms; cyano groups; nitro groups; silyl groups such as trimethylsilyl and triphenylsilyl groups; linear or branched alkyl groups having 1 to 6 carbon atoms such as methyl, ethyl, and propyl groups; linear or branched alkoxy groups having 1 to 6 carbon atoms such as methoxy, ethoxy, and propoxy groups; alkenyl groups such as vinyl and allyl groups; aryloxy groups such as phenyloxy and tolyloxy groups; arylalkyloxy groups such as benzyloxy and phenethyloxy groups; and aryl groups having 6 to 30 carbon atoms or heteroaryl groups having 2 to 20 carbon atoms such as phenyl, biphenylyl, terphenylyl, naphthyl, anthryl, phenanthryl, fluorenyl, spirobifluorenyl, indenyl, pyrenyl, perylenyl, fluoranthenyl, triphenylenyl, pyridyl, thienyl, furyl, pyrrolyl, quinolyl, isoquinolyl, benzofuryl, benzothienyl, indolyl, carbazolyl, benzoxazolyl, benzothiazolyl, quinoxalinyl, quinazolinyl, benzimidazolyl, pyrazolyl, dibenzofuryl, dibenzothienyl, carbolinyl, and phenanthrolinyl groups. These substituents may be further substituted with the substituents exemplified above. These substituents and the benzene ring to which the substituents are bonded, or a plurality of substituents bonded to the same benzene ring, may be bonded to each other to form a ring via a single bond, a substituted or unsubstituted methylene group, an oxygen atom, or a sulfur atom.

In the substituted or unsubstituted phenyl groups represented by B and C, a cyano group is preferred among those exemplified above as a substituent that phenyl groups may have. That is, when B and/or C is a substituted phenyl group, the substituted phenyl group is preferably a cyanophenyl group, and more preferably a 3-cyanophenyl group or a 4-cyanophenyl group.

In the substituted or unsubstituted naphthyl group, the naphthyl group is preferably a 2-naphthyl group. That is, the substituted or unsubstituted naphthyl group is preferably a substituted or unsubstituted 2-naphthyl group. In the substituted or unsubstituted quinolyl group, the quinolyl group is preferably a 2-quinolyl group or 3-quinolyl group. In the substituted or unsubstituted quinoxalinyl group, the quinoxalinyl group is preferably a 2-quinoxalinyl group or 6-quinoxalinyl group. In the substituted or unsubstituted quinazolinyl group, the quinazolinyl group is preferably a 2-quinazolinyl group or 6-quinazolinyl group. In the substituted or unsubstituted phenanthrolinyl group, the phenanthrolinyl group is preferably a [1,10]phenanthrolinyl group and more preferably a 2-[1,10]phenanthrolinyl group. In the substituted or unsubstituted benzoxazolyl group, the benzoxazolyl group is preferably a 2-benzoxazolyl group or 6-benzoxazolyl group and more preferably a 2-benzoxazolyl group. In the substituted or unsubstituted benzothiazolyl group, the benzothiazolyl group is preferably a 2-benzothiazolyl group or 6-benzothiazolyl group, and more preferably a 2-benzothiazolyl group. In the substituted or unsubstituted benzofuryl group, the benzofuryl group is preferably a 2-benzofuryl group. In the substituted or unsubstituted benzothienyl group, the benzothienyl group is preferably a 2-benzothienyl group. In the substituted or unsubstituted oxazolopyridyl group, the oxazolopyridyl group is preferably an oxazolo[5,4-b]pyridyl group, more preferably a 2-oxazolo[5,4-b]pyridyl group or a 5-oxazolo[5,4-b]pyridyl group, and even more preferably a 2-oxazolo[5,4-b]pyridyl group.

B and C each independently are preferably selected from, for example, the following groups, each of which may be substituted or unsubstituted: a phenyl group, 2-naphthyl group, 2-quinolyl group, 3-quinolyl group, 2-quinoxalinyl group, 6-quinoxalinyl group, 2-quinazolinyl group, 6-quinazolinyl group, 2-[1,10]phenanthrolinyl group, 2-benzoxazolyl group, 6-benzoxazolyl group, 2-benzothiazolyl group, 6-benzothiazolyl group, 2-benzofuryl group, 2-benzothienyl group, 2-oxazolo[5,4-b]pyridyl group, or 5-oxazolo[5,4-b]pyridyl group. Furthermore, B and C each independently are more preferably selected from, for example, the following groups, each of which may be substituted or unsubstituted: a phenyl group, 2-naphthyl group, 2-quinolyl group, 3-quinolyl group, 2-quinoxalinyl group, 6-quinoxalinyl group, 2-quinazolinyl group, 6-quinazolinyl group, 2-[1,10]phenanthrolinyl group, 2-benzoxazolyl group, 2-benzothiazolyl group, 2-benzofuryl group, 2-benzothienyl group, or 2-oxazolo[5,4-b]pyridyl group.

In addition, at least one of B and C is preferably selected from the following unsubstituted groups: a phenyl group, naphthyl group, quinolyl group, quinazolinyl group, phenanthrolinyl group, benzoxazolyl group, benzothiazolyl group, or oxazolopyridyl group; at least one of B and C is more preferably selected from the following unsubstituted groups: a phenyl group, naphthyl group, quinolyl group, quinazolinyl group, phenanthrolinyl group, benzothiazolyl group, or oxazolopyridyl group; and at least one of B and C is even more preferably selected from the following unsubstituted groups: a phenyl group, naphthyl group, quinazolinyl group, phenanthrolinyl group, or oxazolopyridyl group.

In general formula (1), D represents a hydrogen atom, a substituted or unsubstituted aryl group, or a substituted or unsubstituted heteroaryl group. Specific examples of the "aryl group" or "heteroaryl group" in the "substituted or unsubstituted aryl group" or the "substituted or unsubstituted heteroaryl group" include groups selected from aryl groups having 6 to 30 carbon atoms or heteroaryl groups having 2 to 20 carbon atoms, such as a phenyl group, biphenylyl group, terphenylyl group, naphthyl group, anthryl group, phenanthryl group, fluorenyl group, spirobifluorenyl group, indenyl group, pyrenyl group, perylenyl group, fluoranthenyl group, triphenylenyl group, pyridyl group, pyrimidinyl group, triazinyl group, thienyl group, furyl group, pyrrolyl group, quinolyl group, isoquinolyl group, benzofuryl group, benzothienyl group, indolyl group, carbazolyl group, benzoxazolyl group, benzothiazolyl group, oxazolopyridyl group, oxazolopyrazyl group, quinoxalinyl group, quinazolinyl group, benzimidazolyl group, pyrazolyl group, dibenzofuryl group, dibenzothienyl group, naphthyridinyl group, phenanthrolinyl group, acridinyl group, or carbolinyl group. The oxazolopyridyl group may be an oxazolo[4,5-b]pyridyl group or an oxazolo[5,4-b]pyridyl group.

Examples of "substituents" that the "aryl group" or "heteroaryl group" in the substituted or unsubstituted aryl group or heteroaryl group represented by D may have include the same substituents as those exemplified above as substituents that the respective groups represented by B and C above may have, and the same applies to the embodiments of the substituents. The substituted or unsubstituted aryl group or heteroaryl group represented by D is preferably a cyanophenyl group (a phenyl group having a cyano group as a substituent), an unsubstituted aryl group, or an unsubstituted heteroaryl group.

In addition, the substituted or unsubstituted aryl group or heteroaryl group represented by D is preferably selected from the group consisting of a substituted or unsubstituted phenyl group, biphenylyl group, naphthyl group, furyl group, quinolyl group, benzoxazolyl group, benzothiazolyl group, oxazolopyridyl group, quinoxalinyl group, quinazolinyl group, and phenanthrolinyl group, and is more preferably selected from the group consisting of a cyanophenyl group (a phenyl group having a cyano group as a substituent) as well as an unsubstituted phenyl group, biphenylyl group, naphthyl group, furyl group, quinolyl group, benzoxazolyl group, benzothiazolyl group, oxazolopyridyl group, quinoxalinyl group, quinazolinyl group, and phenanthrolinyl group. That is, D is preferably a hydrogen atom or selected from the following substituted or unsubstituted groups: a phenyl group, biphenylyl group, naphthyl group, furyl group, quinolyl group, benzoxazolyl group, benzothiazolyl group, oxazolopyridyl group, quinoxalinyl group, quinazolinyl group, or phenanthrolinyl group. Further, D is more preferably a hydrogen atom, a cyanophenyl group, or selected from the following unsubstituted groups: a phenyl group, biphenylyl group, naphthyl group, furyl group, quinolyl group, benzoxazolyl group, benzothiazolyl group, oxazolopyridyl group, quinoxalinyl group, quinazolinyl group, or phenanthrolinyl group.

L₁ to L₃ each independently represent either a single bond or an unsubstituted divalent aryl group or an unsubstituted divalent heteroaryl group. The "divalent aryl group" or "divalent heteroaryl group" in the "unsubstituted divalent aryl group" or "unsubstituted divalent heteroaryl group" include the divalent groups obtained by removing one hydrogen atom from the "aryl group" or "heteroaryl group" represented by D described above, for example, a divalent group obtained by removing one hydrogen atom from the specific groups exemplified above. The "divalent aryl group" is preferably a phenylene group, a biphenylylene group, or a naphthylene group.

L₁ to L₃ each independently are preferably either a single bond or an unsubstituted phenylene group, an unsubstituted biphenylylene group, or an unsubstituted naphthylene group, and more preferably a single bond, an unsubstituted 1,3-phenylene group or 1,4-phenylene group, an unsubstituted 4,4'-biphenylylene group, an unsubstituted 2,6-naphthylene group, or an unsubstituted 2,7-naphthylene group. In addition, L₁ and L₃ are preferably the same bond or group, and more preferably, L₁ and L₃ are both single bonds or both phenylene groups (preferably a 1,3-phenylene group or a 1,4-phenylene group).

Of the benzazole compounds represented by the above general formula (1) applied to the capping layer of the organic EL device of the present invention, the benzazole compounds represented by the following general formula (2) are novel compounds and have a more characteristic structure. The benzazole compounds represented by the above general formula (1) may be benzazole compounds represented by the following general formula (2):

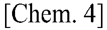

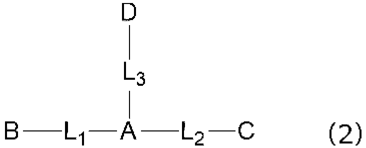

In general formula (2), A represents a benzoxazolyl ring or benzothiazolyl ring and is bonded to L₁, L₂, and L₃ on the carbon atoms constituting the ring. A is preferably a benzoxazolyl ring. B and C each independently represent a substituted or unsubstituted group selected from the following groups: a phenyl group, naphthyl group, quinolyl group, quinoxalinyl group, quinazolinyl group, phenanthrolinyl group, benzoxazolyl group, benzothiazolyl group, benzofuryl group, benzothienyl group, or oxazolopyridyl group. D represents either a hydrogen atom or a group selected from the following groups, each of which may be substituted or unsubstituted: a phenyl group, biphenylyl group, naphthyl group, furyl group, quinolyl group, quinoxalinyl group, quinazolinyl group, phenanthrolinyl group, benzoxazolyl group, benzothiazolyl group, benzofuryl group, benzothienyl group, or oxazolopyridyl group. L₁ to L₃ represent, independently of each other, either a single bond or an unsubstituted phenylene group, biphenylylene group, or naphthylene group. In general formula (2), L₂, L₃, and L₁ are bonded to the 2-position, 4-position, and 6-position, respectively, of the benzoxazolyl ring or benzothiazolyl ring represented by A.

In general formula (2), either of the following requirements 1 or 2 shall be satisfied:
Requirement 1: At least one of B, C, and D contains a structure selected from a group consisting of quinazoline, phenanthroline, and oxazolopyridine, provided that:
   if one or two of B, C, and D are substituted or unsubstituted phenanthrolinyl groups and none of the one or two of B, C, and D that are not the phenanthrolinyl groups is a group selected from a substituted or unsubstituted quinazolinyl group and oxazolopyridyl group, then at least one of L₁, L₂, and L₃ bonded to the phenanthrolinyl groups is an unsubstituted phenylene group, biphenylylene group, or naphthylene group (i.e., not a single bond); and
   if B, C, and D are all substituted or unsubstituted phenanthrolinyl groups, at least one selected from L₁, L₂, and L₃ bonded to the phenanthrolinyl groups is an unsubstituted phenylene group, biphenylylene group, or naphthylene group (i.e., not a single bond); and
Requirement 2: None of B, C, and D contains any structure selected from a group consisting of quinazoline, phenanthroline, and oxazolopyridine, L₃ is a single bond, and D is a hydrogen atom.

In other words, in Requirement 1, at least one of B, C, and D contains a structure selected from a group consisting of quinazoline, phenanthroline, and oxazolopyridine, except in a case where (1) at least one of B, C, and D is a phenanthrolinyl group and any of the remaining B, C, and D are neither a quinazolinyl group nor an oxazolopyridyl group, and at least one selected from L₁, L₂, and L₃ bonded to the phenanthrolinyl group is entirely a single bond. Further, (2) in a case where B, C, and D are all substituted or unsubstituted phenanthrolinyl groups, a case where all of L₁-B, L₂-C, and L₃-D are single bond-phenanthrolinyl groups is excluded, and at least one of L₁, L₂, and L₃ must be a phenylene group, biphenylylene group, or naphthylene group and not a single bond.

Here, L₁, L₂, and L₃ bonded to the structure selected from a group consisting of quinazoline and phenanthroline are preferably not single bonds. That is, when at least one of B, C, and D is a quinazoline structure orand phenanthroline structure, L₁, L₂, and L₃ bonded to any of such structures are each independently preferably an unsubstituted phenylene group, biphenylylene group, or naphthylene group. In general formula (2), in particular, at least one of B, C, and D preferably contains an oxazolopyridine structure. In this case, L₁, L₂, and L₃ bonded to the oxazolopyridine structure may be single bonds. The oxazolopyridine may be an oxazolo[4,5-b]pyridine or an oxazolo[5,4-b]pyridine, and is preferably oxazolo[5,4-b]pyridine.

In general formula (2), at least one of B and C is preferably a group selected from the group consisting of substituted or unsubstituted phenyl, naphthyl, quinolyl, quinazolinyl, phenanthrolinyl, benzoxazolyl, benzothiazolyl, benzofuryl, and oxazolopyridyl groups. At least one of B and C is more preferably a group selected from the group consisting of unsubstituted phenyl, naphthyl (preferably 2-naphthyl), quinolyl (preferably 2-quinolyl or 3-quinolyl), quinazolinyl (preferably 2-quinazolinyl or 6-quinazolinyl), phenanthrolinyl (preferably 2-[1,10]-phenanthrolinyl), benzoxazolyl (preferably 2-benzoxazolyl or 6-benzoxazolyl), benzothiazolyl (preferably 2-benzothiazolyl or 6-benzothiazolyl), benzofuryl (preferably 2-benzofuryl), and oxazolopyridyl (preferably 2-oxazolo[5,4-b]pyridyl or 5-oxazolo[5,4-b]pyridyl, and more preferably 2-oxazolo[5,4-b]pyridyl). At least one of B and C is even more preferably a group selected from the group consisting of unsubstituted phenyl, naphthyl, quinolyl, quinazolinyl, phenanthrolinyl, benzothiazolyl, and oxazolopyridyl. Regarding details of A to D and L₁ to L₃ in general formula (2), reference may be made as appropriate to the description of general formula (1) above.

The benzazole compounds represented by the above general formula (1) or (2) can be synthesized, for example, in accordance with known methods, as shown below. In addition, these compounds can be synthesized by known coupling reactions using, for example, a palladium catalyst (see, for example, Patent Document 2 and Non-Patent Documents 4 and 5).

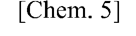

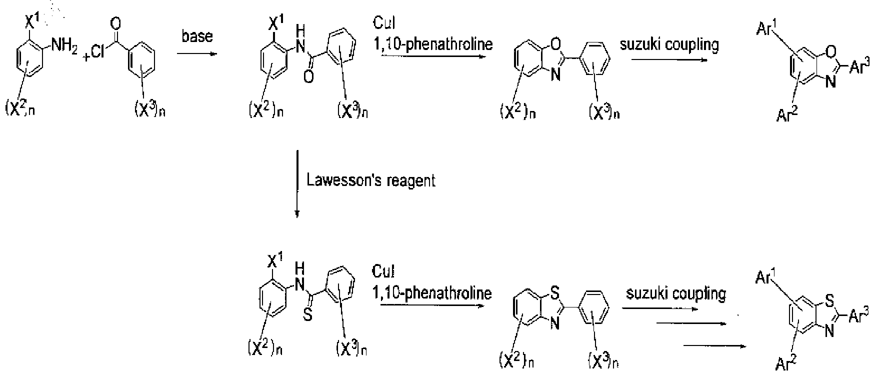

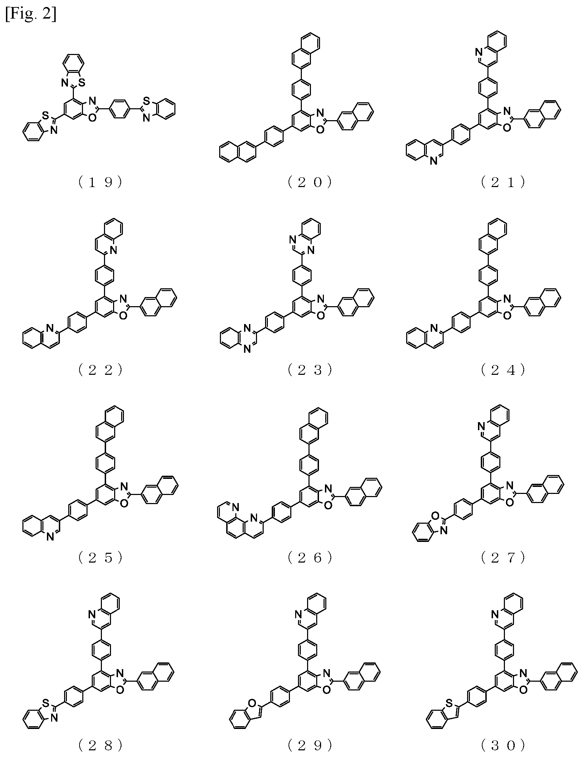

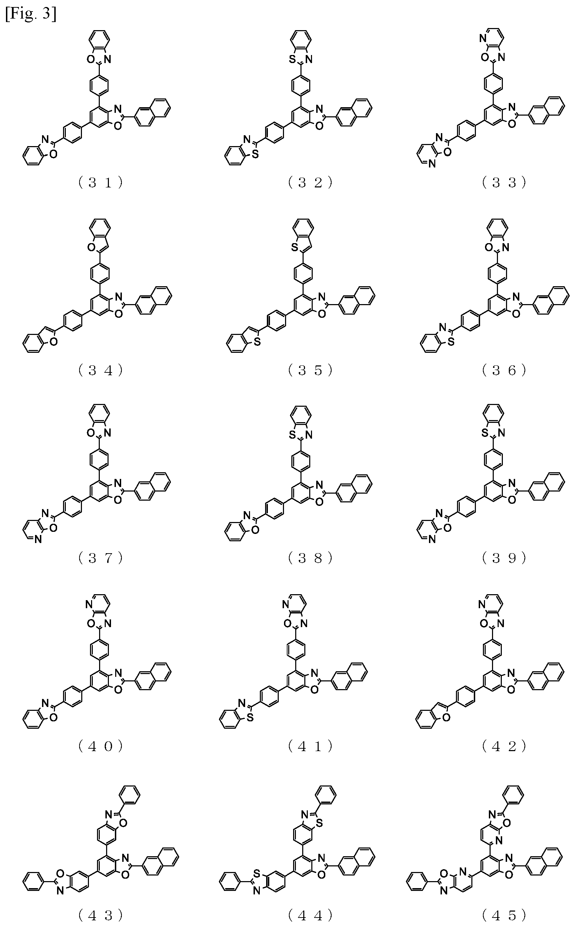

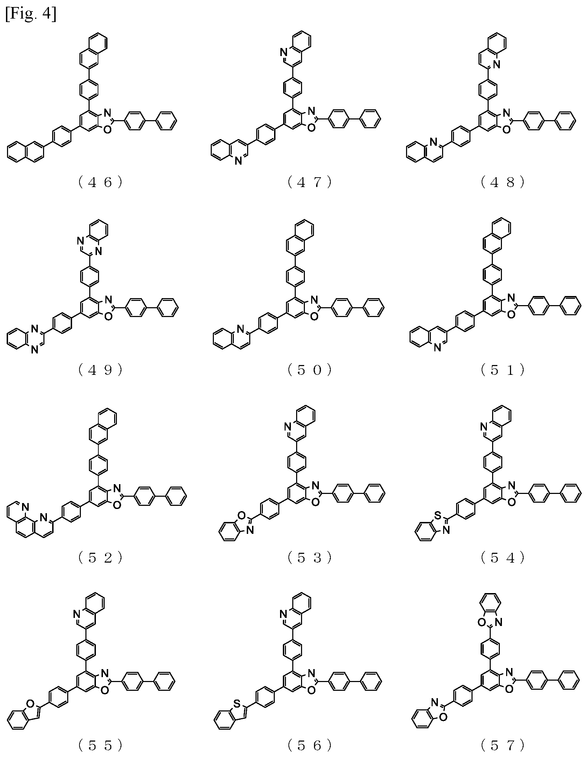

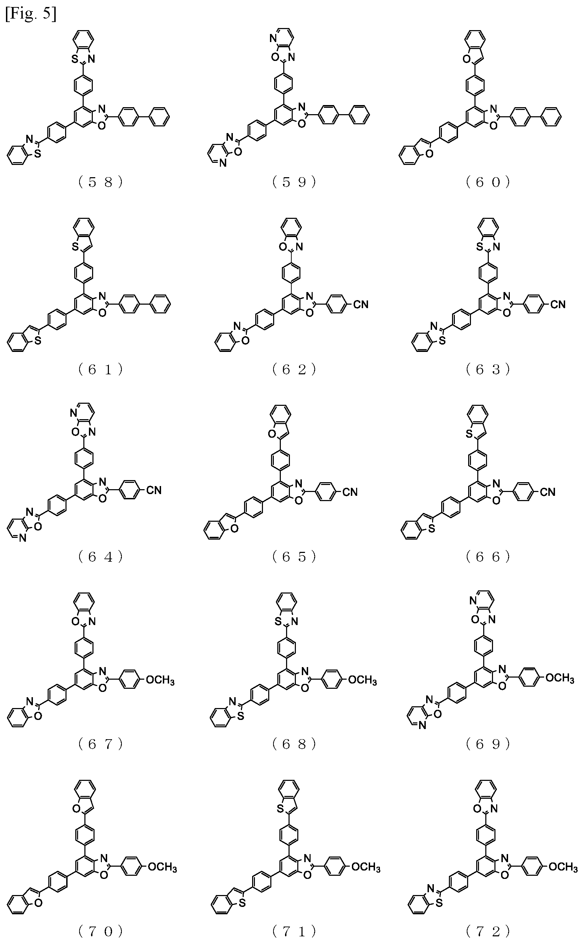

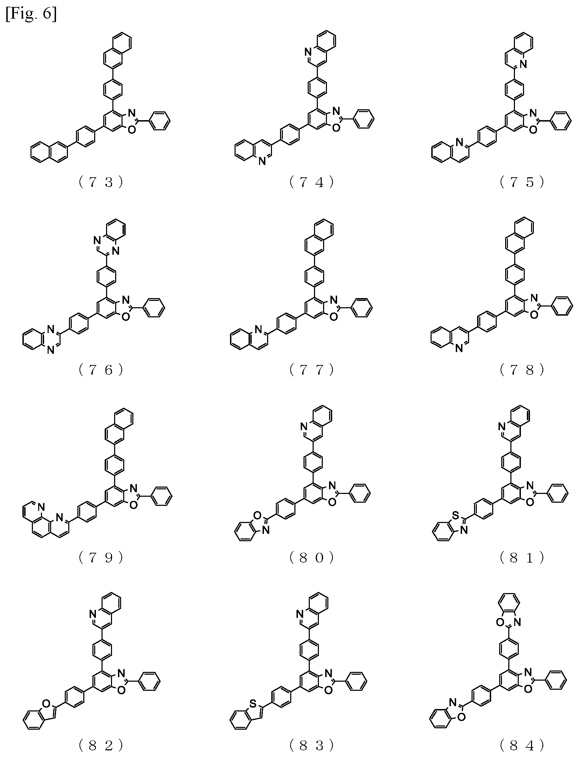

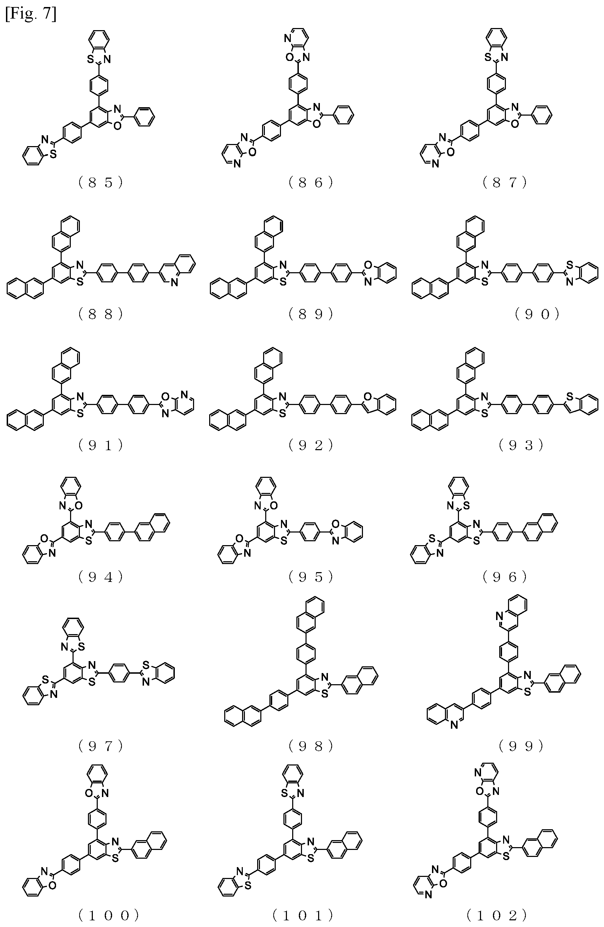

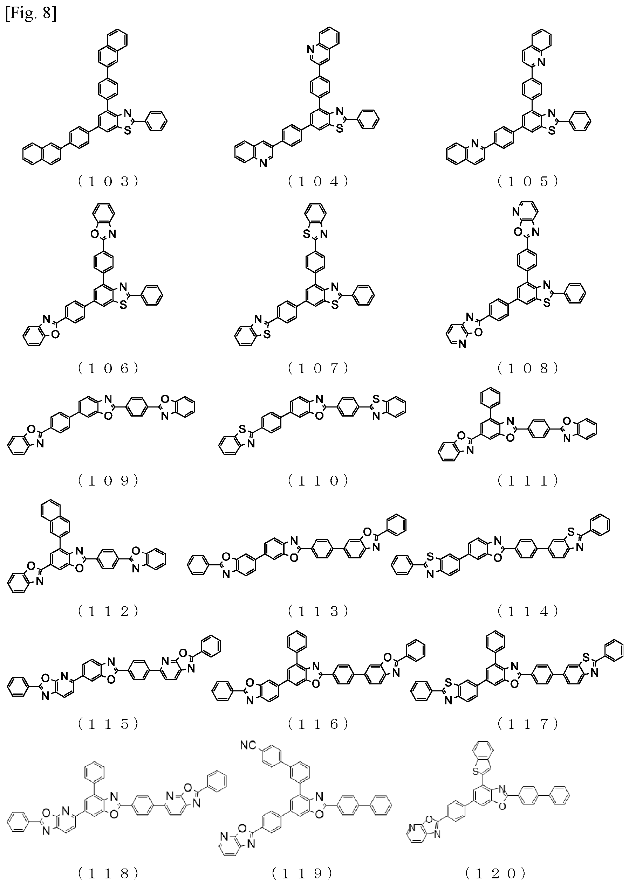

Specific examples of preferred compounds among the benzazole compounds represented by general formula (1) or (2) are shown in Figs. 1 to 9. However, the present invention is not limited to these compounds.

The method for purifying the benzazole compounds is not particularly limited, and the compounds can be purified by known methods used for the purification of organic compounds such as: purification by column chromatography; adsorption purification using silica gel, activated carbon, activated clay, or the like; recrystallization purification or crystallization purification using a solvent; and sublimation purification. The compounds can be identified, for example, by NMR analysis.

As physical property values of the benzazole compounds, the melting point, glass transition point (Tg), refractive index, and extinction coefficient are preferably measured. The melting point serves as an index of vapor deposition properties and the glass transition point serves as an index of stability in a thin-film state. In addition, the refractive index and extinction coefficient are indicators of improvement in light extraction efficiency.

The melting point and glass transition point can be measured on powder samples using a high-sensitivity differential scanning calorimeter (for example, trade name: DSC3100SA manufactured by Bruker AXS). The refractive index and extinction coefficient can be measured for a thin film formed to a thickness of 80 nm on a silicon substrate using a spectrometer (for example, trade name: F10-RT-UV manufactured by Filmetrics).

In the present invention, the capping layer contains a benzazole compound represented by general formula (1). Among the benzazole compounds represented by general formula (1), the benzazole compounds represented by general formula (2) are more preferable. The capping layer may also be a laminated layer or a mixed layer containing two or more kinds of compounds including the benzazole compounds represented by general formula (1) above. Examples of compounds other than the benzazole compounds represented by general formula (1) include, for example, phthalic acid derivatives (see Patent Document 3). Of course, the capping layer may contain two or more kinds of benzazole compounds represented by the above general formula (1). These materials may be individually formed into a film or may be used as a single layer formed by mixing with other materials. Alternatively, the capping layer may have a laminated structure composed by combining individually formed layers, by combining layers formed by mixing with other materials, or by combining individually formed layers with layers formed by mixing with other materials. These materials can be formed into films by known methods such as vapor deposition, spin coating, and inkjet methods.

In the present invention, when a capping layer that contains a benzazole compound represented by the above general formula (1) or (2) is formed to a thickness of 30 nm to 120 nm, the refractive index of the formed film is preferably 1.70 or more in a wavelength range of 450 nm to 700 nm. The refractive index is more preferably 1.85 or more, and even more preferably 1.90 or more.

### Other Respective Layers

As materials for each of the other layers, known materials may be appropriately selected and used. The substrate is not particularly limited, and a glass substrate, a plastic substrate, or the like may be used. As materials for the anode, an electrode material having a large work function such as ITO or gold may be used.

Materials that can be used for the hole injection layer include arylamine compounds having a structure in which two or more triphenylamine structures are joined within a molecule via a single bond or a divalent group that does not contain a heteroatom such as starburst-type triphenylamine derivatives, various triphenylamine tetramers, porphyrin compounds represented by copper phthalocyanine, and acceptor-type heterocyclic compounds such as hexacyanoazatriphenylene, as well as coating-type polymer materials. These materials may be individually formed into a film or may be used as a single layer formed by mixing with other materials. Alternatively, the hole injection layer may have a laminated structure composed by combining individually formed layers, by combining layers formed by mixing with other materials, or by combining individually formed layers with layers formed by mixing with other materials. These materials can be formed into films by known methods such as vapor deposition, spin coating, and inkjet methods.

Materials that can be used for the hole transport layer include benzidine derivatives such as N,N'-diphenyl-N,N'-di(m-tolyl)benzidine (TPD), N,N'-diphenyl-N,N'-di(α-naphthyl)benzidine, and N,N,N',N'-tetrabiphenylylbenzidine, as well as 1,1-bis[4-(di-4-tolylamino)phenyl]cyclohexane. In particular, arylamine compounds having a structure in which two triphenylamine structures are joined within a molecule via a single bond or a divalent group that does not contain a heteroatom such as N,N,N',N'-tetrabiphenylbenzidine are preferred. Arylamine compounds having a structure in which three or more triphenylamine structures are joined within a molecule via a single bond or a divalent group that does not contain a heteroatom such as various triphenylamine trimers and tetramers are also preferably used. These materials may be individually formed into a film or may be used as a single layer formed by mixing with other materials. Alternatively, the hole transport layer may have a laminated structure composed by combining individually formed layers, by combining layers formed by mixing with other materials, or by combining individually formed layers with layers formed by mixing with other materials. In addition, a coating-type polymer material such as poly(3,4-ethylenedioxythiophene)/poly(styrene sulfonate) may be used as the material for the hole injection layer and hole transport layer. These materials can be formed into films by known methods such as vapor deposition, spin coating, and inkjet methods.

Materials that can be used for the hole injection layer and hole transport layer include p-doped materials in which dopants such as tris(bromophenyl)amine hexachloroantimonate and radialene derivatives (for example, see Patent Document 1) are doped into materials commonly used for these layers. In addition, polymer compounds having a benzidine derivative structure such as TPD as a part of the compound structure may also be used.

The organic EL device of the present invention may also include an electron-blocking layer. Materials that can be used for the electron-blocking layer include carbazole derivatives such as 4,4',4"-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-yl-phenyl)adamantane, and compounds having a triphenylsilyl group and a triarylamine structure represented by 9-[4-(carbazol-9-yl)phenyl]-9-[4-(triphenylsilyl)phenyl]-9H-fluorene, both of which have an electron-blocking function. These materials may be individually formed into a film or may be used as a single layer formed by mixing with other materials. Alternatively, the electron-blocking layer may have a laminated structure composed by combining individually formed layers, by combining layers formed by mixing with other materials, or by combining individually formed layers with layers formed by mixing with other materials. These materials can be formed into films by known methods such as vapor deposition, spin coating, and inkjet methods.

Materials that can be used for the light-emitting layer include metal complexes of quinolinol derivatives such as Alq₃, various metal complexes, anthracene derivatives, bis-styrylbenzene derivatives, pyrene derivatives, oxazole derivatives, and poly(p-phenylene vinylene) derivatives. The light-emitting layer may also have a structure containing a host material and a dopant material. Anthracene derivatives are preferably used as the host material. Materials that can be used as the host materials include the materials of the aforementioned light-emitting layer, heterocyclic compounds having an indole ring as a fused ring substructure, heterocyclic compounds having a carbazole ring as a fused ring substructure, carbazole derivatives, thiazole derivatives, benzimidazole derivatives, and polydialkylfluorene derivatives. As the dopant materials, quinacridone, coumarin, rubrene, perylene, pyrene and their derivatives, benzopyran derivatives, rhodamine derivatives, aminostyryl derivatives, and the like can be used. Green-light-emitting materials are preferred as the dopant materials. These materials may be individually formed into a film or may be used as a single layer formed by mixing with other materials. Alternatively, the light-emitting layer may have a laminated structure composed by combining individually formed layers, by combining layers formed by mixing with other materials, or by combining individually formed layers with layers formed by mixing with other materials.

Phosphorescent emitters can also be used as the dopant materials. As an example, metal complexes of iridium or platinum can be used as the phosphorescent emitters. Examples of the phosphorescent emitters include green phosphorescent emitters such as Ir(ppy)₃, blue phosphorescent emitters such as FIrpic and FIr6, and red phosphorescent emitters such as Btp₂Ir(acac). Among these, green phosphorescent emitters are preferably used. When a phosphorescent emitter is used as the dopant material, host materials having hole-injecting and hole-transporting properties such as carbazole derivatives including 4,4'-di(N-carbazolyl)biphenyl, TCTA, and mCP, or host materials having electron-transporting properties such as p-bis(triphenylsilyl)benzene and 2,2',2"-(1,3,5-phenylene)tris(1-phenyl-1H-benzimidazole) can be used as the host material. Doping of the phosphorescent emitters into the host material is preferably performed by co-deposition in a range of 1 to 30 wt% with respect to the whole light-emitting layer to avoid concentration quenching.

In addition, materials that emit delayed fluorescence such as CDCB derivatives exemplified by PIC-TRZ, CC2TA, PXZ-TRZ, and 4CzIPN can also be used as the dopant material (for example, see Non-Patent Document 6). These materials can be formed into films by known methods such as vapor deposition, spin coating, and inkjet methods.

The organic EL device of the present invention may include a hole-blocking layer. Materials that can be used for the hole-blocking layer include compounds having hole-blocking properties such as phenanthroline derivatives exemplified by bathocuproine, metal complexes of quinolinol derivatives such as aluminum(III) bis(2-methyl-8-quinolinate)-4-phenylphenolate (BAlq), various rare-earth complexes, triazole derivatives, triazine derivatives, pyrimidine derivatives, oxadiazole derivatives, and benzazole derivatives. These materials may also be used as materials for the electron transport layer. These materials may be individually formed into a film or may be used as a single layer formed by mixing with other materials. Alternatively, the hole-blocking layer may have a laminated structure composed by combining individually formed layers, by combining layers formed by mixing with other materials, or by combining individually formed layers with layers formed by mixing with other materials. These materials can be formed into films by known methods such as vapor deposition, spin coating, and inkjet methods.

Materials that can be used for the electron transport layer include metal complexes of quinolinol derivatives such as Alq₃ and BAlq, various metal complexes, triazole derivatives, triazine derivatives, pyrimidine derivatives, oxadiazole derivatives, pyridine derivatives, benzimidazole derivatives, benzazole derivatives, thiadiazole derivatives, anthracene derivatives, carbodiimide derivatives, quinoxaline derivatives, pyridoindole derivatives, phenanthroline derivatives, and silole derivatives. These materials may be individually formed into a film or may be used as a single layer formed by mixing with other materials. Alternatively, the electron transport layer may have a laminated structure composed by combining individually formed layers, by combining layers formed by mixing with other materials, or by combining individually formed layers with layers formed by mixing with other materials. These materials can be formed into films by known methods such as vapor deposition, spin coating, and inkjet methods.

Materials that can be used for the electron injection layer include alkali metal salts such as lithium fluoride and cesium fluoride, alkaline earth metal salts such as magnesium fluoride, metal complexes of quinolinol derivatives such as lithium quinolinolate, metal oxides such as aluminum oxide, and metals such as ytterbium (Yb), samarium (Sm), calcium (Ca), strontium (Sr), and cesium (Cs). The electron injection layer may be omitted depending on the preferred combination of the electron transport layer and the cathode.

Furthermore, materials that can be used for the electron transport layer and the electron injection layer include N-doped materials in which metals such as cesium are doped into the materials commonly used for these layers.

Materials that can be used for the cathode include metals having a low work function such as aluminum, alloys having an even lower work function such as magnesium-silver alloy, magnesium-calcium alloy, magnesium-indium alloy, and aluminum-magnesium alloy, and in addition, ITO and IZO.

The total thickness of each layer of the organic EL device is preferably from 200 nm to 750 nm, and more preferably from 350 nm to 600 nm. The thickness of the capping layer is preferably, for example, 30 nm to 120 nm, and more preferably 40 nm to 80 nm. When the thickness of the capping layer is within the above range, improved light extraction efficiency can be obtained. The thickness of the capping layer can be adjusted as appropriate depending on the type of light-emitting material used in the light-emitting device and the thicknesses of each layer other than the capping layer.

Although the above description relates to an organic EL device having a top-emission structure, the present invention is not limited thereto. The invention can similarly be applied to an organic EL device having a bottom-emission structure or a dual-emission structure that emits light from both the top and bottom. In any case, the electrode located on the side from which light is extracted from the light-emitting device is preferably transparent or semi-transparent.

The physical properties of compounds suitable for the capping layer of the organic EL device include (1) high refractive index, (2) low extinction coefficient, (3) ability to be vapor-deposited, (4) stability in a thin-film state, and (5) high glass transition temperature. Furthermore, the physical properties of the organic EL device include (1) high light extraction efficiency, (2) suppression of color purity degradation, (3) transmission of light without temporal changes, and (4) long lifetime. The benzazole compounds of the present invention represented by general formula (1) or (2) have a high refractive index, a low extinction coefficient, can be vapor-deposited, are stable in a thin-film state, and have a high glass transition temperature. The use of such benzazole compounds in the capping layer therefore enables the provision of an organic EL device having high light extraction efficiency, suppressed color purity degradation, light transmission without temporal changes, and long lifetime.

### Electronic Apparatus or Electronic Device

The electronic apparatus or electronic device of the present invention includes a pair of electrodes and at least one organic layer, wherein the organic layer contains a benzazole compound represented by the above general formula (1) or (2). Preferably, at least one of the organic layers is provided on the outer side of one of the electrodes, and more preferably, the organic layer provided on the outer side of the electrode contains a benzazole compound represented by general formula (1) or (2).

### EXAMPLES

Although the following examples specifically illustrate embodiments of the present invention, the present invention is not limited to the following examples as long as embodiments do not depart from the gist of the invention.

### Example 1: Synthesis of Compound (4)

Amounts of 8.8 g of 2-(4-chlorophenyl)-4,6-di(naphthalen-2-yl)benzoxazole, 6.7 g of 3-[4-(4,4,5,5-tetramethyl-1,3,2-dioxaborolan-2-yl)phenyl]quinoline, 0.5 g of tris(dibenzylideneacetone)dipalladium(0), 0.5 g of tricyclohexylphosphine, and 7.8 g of tripotassium phosphate were charged into a reaction vessel, and the mixture was stirred under reflux overnight in a mixed solvent of 1,4-dioxane/H₂O. After the resulting mixture was allowed to cool, MeOH was added and the precipitated solid was filtered to obtain a crude product. The obtained crude product was purified by recrystallization using monochlorobenzene, whereby 5.0 g of 4,6-di(naphthalen-2-yl)-2-{4'-(quinolin-3-yl)[1,1'-biphenyl]-4-yl}benzoxazole (Compound (4)) was obtained as a pale yellow powder (yield: 42.0%).

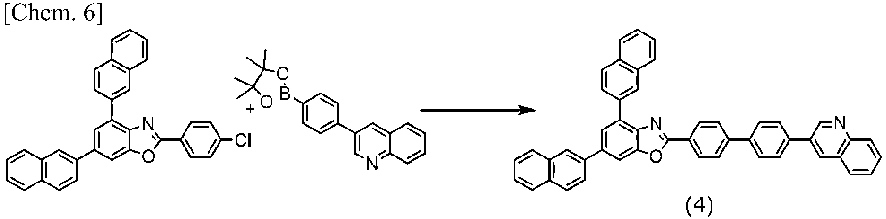

The structure of the obtained pale yellow powder was identified by NMR. In the ¹H-NMR (DMSO-d₆), the following 30 proton signals were detected:
δ (ppm) = 9.37 (1H), 8.83 (1H), 8.77 (1H), 8.52 (1H), 8.45 (1H), 8.42 (2H), 8.30 (2H), 8.19-7.99 (15H), 7.81 (1H), 7.68 (1H), and 7.60 (4H).

### Example 2: Synthesis of Compound (12)

Amounts of 11.3 g of 2-(4-chlorophenyl)-4,6-di(quinolin-3-yl)benzoxazole, 6.4 g of 2-[4-(4,4,5,5-tetramethyl-1,3,2-dioxaborolan-2-yl)phenyl]naphthalene, 0.6 g of tris(dibenzylideneacetone)dipalladium(0), 0.7 g of tricyclohexylphosphine, and 9.9 g of tripotassium phosphate were charged into a reaction vessel, and the mixture was stirred under reflux overnight in a mixed solvent of 1,4-dioxane/H₂O. After the resulting mixture was allowed to cool, MeOH was added and the precipitated solid was filtered to obtain a crude product. The obtained crude product was purified by recrystallization using monochlorobenzene, whereby 6.3 g of 2-{4'-(naphthalen-2-yl)[1,1'-biphenyl]-4-yl}-4,6-di(quinolin-3-yl)benzoxazole (Compound (12)) was obtained as a pale yellow powder (yield: 41.3%).

The structure of the obtained pale yellow powder was identified by NMR. In the ¹H-NMR (DMSO-d₆), the following 29 proton signals were detected:
δ (ppm) = 9.84 (1H), 9.54 (1H), 9.24 (1H), 8.96 (1H), 8.47 (2H), 8.42 (2H), 8.21 (1H), 8.23-7.92 (14H), 7.88 (1H), 7.83 (1H), 7.72 (2H), and 7.57 (2H).

### Example 3: Synthesis of Compound (32)

Amounts of 8.0 g of 4,6-dibromo-2-(naphthalen-2-yl)benzoxazole, 14.1 g of 2-[4-(4,4,5,5-tetramethyl-1,3,2-dioxaborolan-2-yl)phenyl]benzothiazole, 0.9 g of tetrakis(triphenylphosphine)palladium(0), and 11.0 g potassium carbonate were charged into a reaction vessel, and the mixture was stirred under reflux overnight in a mixed solvent of toluene/EtOH/H₂O. After the resulting mixture was allowed to cool, MeOH was added and the precipitated solid was filtered to obtain a crude product. The obtained crude product was purified by recrystallization using monochlorobenzene, whereby 8.7 g of 4,6-bis(4-benzothiazol-2-yl-phenyl)-2-(naphthalen-2-yl)-benzoxazole (Compound (32)) was obtained as a pale yellow powder (yield: 66.0%).

The structure of the obtained pale yellow powder was identified by NMR. In the ¹H-NMR (DMSO-d₆), the following 25 proton signals were detected:
δ (ppm) = 8.93 (1H), 8.51 (2H), 8.39 (1H), 8.37 (1H), 8.34 (2H), 8.30-8.06 (12H), 7.70 (2H), 7.61 (2H), and 7.52 (2H).

### Example 4: Synthesis of Compound (33)

Amounts of 4.3 g of 4,6-dibromo-2-(naphthalen-2-yl)benzoxazole, 7.6 g of 2-[4-(4,4,5,5-tetramethyl-1,3,2-dioxaborolan-2-yl)phenyl]oxazolo[5,4-b]pyridine, 0.5 g of tetrakis(triphenylphosphine)palladium(0), and 4.4 g of potassium carbonate were charged into a reaction vessel, and the mixture was stirred under reflux overnight in a mixed solvent of toluene/EtOH/H₂O. After the resulting mixture was allowed to cool, MeOH was added and the precipitated solid was filtered to obtain a crude product. The obtained crude product was purified by recrystallization using 1,2-dichlorobenzene, whereby 5.2 g of 2-(naphthalen-2-yl)-4,6-bis(4-oxazolo[4,5-b]pyridin-2-yl-phenyl)-benzoxazole (Compound (33)) was obtained as a pale yellow powder (yield: 76.9%).

The structure of the obtained pale yellow powder was identified by NMR. In the ¹H-NMR (THF-d₈), the following 23 proton signals were detected:
δ (ppm) = 8.96 (1H), 8.58 (4H), 8.49 (3H), 8.38 (2H), 8.24 (2H), 8.21-8.10 (6H), 8.02 (1H), 7.65 (2H), and 7.45 (2H).

### Example 5: Synthesis of Compound (58)

Amounts of 5.0 g of 4,6-dibromo-2-([1,1'-biphenyl]-4-yl)benzoxazole, 9.2 g of 2-[4-(4,4,5,5-tetramethyl-1,3,2-dioxaborolan-2-yl)phenyl]benzothiazole, 0.9 g of tetrakis(triphenylphosphine)palladium(0), and 11.0 g of potassium carbonate were charged into a reaction vessel, and the mixture was stirred under reflux overnight in a mixed solvent of toluene/EtOH/H₂O. After the resulting mixture was allowed to cool, MeOH was added and the precipitated solid was filtered to obtain a crude product. The obtained crude product was purified by recrystallization using monochlorobenzene, whereby 8.9 g of 2-([1,1'-biphenyl]-4-yl)-4,6-bis(4-benzothiazol-2-yl-phenyl)-benzoxazole (Compound (58)) was obtained as a pale yellow powder (yield: 99.2%).

The structure of the obtained pale yellow powder was identified by NMR. In the ¹H-NMR (DMSO-d₆), the following 27 proton signals were detected:
δ (ppm) = 8.50 (2H), 8.39 (2H), 8.33 (3H), 8.26 (2H), 8.23-8.10 (7H), 8.00 (2H), 7.83 (2H), and 7.64-7.44 (7H).

### Example 6: Synthesis of Compound (74)

Amounts of 2.5 g of 4,6-dibromo-2-phenyl-benzoxazole, 4.9 g of 3-[4-(4,4,5,5-tetramethyl-1,3,2-dioxaborolan-2-yl)phenyl]quinoline, 0.4 g of tetrakis(triphenylphosphine)palladium(0), and 2.9 g of potassium carbonate were charged into a reaction vessel, and the mixture was stirred under reflux overnight in a mixed solvent of toluene/EtOH/H₂O. After the resulting mixture was allowed to cool, MeOH/H₂O was added and the precipitated solid was filtered to obtain a crude product. The obtained crude product was purified by recrystallization using monochlorobenzene, whereby 2.8 g of 2-phenyl-4,6-bis(4-quinolin-3-yl-phenyl)-benzoxazole (Compound (74)) was obtained as a white powder (yield: 65.7%).

The structure of the obtained white powder was identified by NMR. In the ¹H-NMR (CDCl₃), the following 27 proton signals were detected:
δ (ppm) = 9.30 (2H), 8.42 (2H), 8.38 (2H), 8.33 (2H), 8.18 (2H), 7.96-7.88 (10H), 7.76 (2H), 7.62 (2H), and 7.58-7.57 (3H).

### Example 7: Synthesis of Compound (86)

Amounts of 5.0 g of 4,6-dibromo-2-phenyl-benzoxazole, 10.0 g of 2-[4-(4,4,5,5-tetramethyl-1,3,2-dioxaborolan-2-yl)phenyl]oxazolo[5,4-b]pyridine, 0.8 g of tetrakis(triphenylphosphine)palladium(0), and 5.9 g of potassium carbonate were charged into a reaction vessel, and the mixture was stirred under reflux overnight in a mixed solvent of toluene/EtOH/H₂O. After the resulting mixture was allowed to cool, H₂O was added and the precipitated solid was filtered to obtain a crude product. The obtained crude product was purified by recrystallization using 1,2-dichlorobenzene, whereby 5.5 g of 2-phenyl-4,6-bis(4-oxazolo[4,5-b]pyridin-2-yl-phenyl)-benzoxazole (Compound (86)) was obtained as a white powder (yield: 66.5%).

The structure of the obtained white powder was identified by NMR. In the ¹H-NMR (CDCl₃), the following 21 proton signals were detected:
δ (ppm) = 8.50 (2H), 8.45 (2H), 8.40-8.36 (6H), 8.12 (2H), 7.95-7.92 (4H), 7.59-7.58 (3H), and 7.41-7.37 (2H).

### Example 8: Synthesis of Compound (119)

Amounts of 9.0 g of 3'-(2-([1,1'-biphenyl]-4-yl)-6-chlorobenzo[b]oxazol-4-yl)-[1,1'-biphenyl]-4-carbonitrile, 6.6 g of 2-{4-(4,4,5,5-tetramethyl-1,3,2-dioxaborolan-2-yl)phenyl}oxazolo[5,4-b]pyridine, 0.5 g of tris(dibenzylideneacetone)dipalladium(0), 0.6 g of tricyclohexylphosphine, and 7.9 g of tripotassium phosphate were charged into a reaction vessel, and the mixture was stirred under reflux overnight in a mixed solvent of 1,4-dioxane/H₂O. After the resulting mixture was allowed to cool, H₂O was added and the precipitated solid was filtered to obtain a crude product. The obtained crude product was purified by recrystallization using 1,2-dichlorobenzene, whereby 7.1 g of 3'-(2-([1,1'-biphenyl]-4-yl)-6-(4-(oxazolo[4,5-b]pyridin-2-yl)phenyl)benzo[b]oxazol-4-yl)-[1,1'-biphenyl]-4-carbonitrile (Compound (119)) was obtained as a yellow powder (yield: 59.3%).

The structure of the obtained yellow powder was identified by NMR. In the ¹H-NMR (CDCl₃), the following 26 proton signals were detected:
δ (ppm) = 8.43 (2H), 8.39 (4H), 8.15 (1H), 8.10 (1H), 7.92-7.88 (4H), 7.85 (2H), 7.79 (4H), 7.70-7.68 (4H), 7.50 (2H), and 7.44-7.37 (2H).

With respect to the compounds obtained in Examples 1 to 8, melting points and glass transition points (Tg) were measured using a high-sensitivity differential scanning calorimeter (trade name: DSC3100SA manufactured by Bruker AXS). The results are shown in Table 1.

**[Table 1]**

| Example | Compound | Melting point (°C) | Glass transition point (°C) |
|---|---|---|---|
| 1 | (4) | 240.2, 255.5 | 104.8 |
| 2 | (12) | 289.8 | 119.9 |
| 3 | (32) | 314.3 | - |
| 4 | (33) | 362.2 | 145.4 |
| 5 | (58) | - | 133.9 |
| 6 | (74) | 308.5 | 109.7 |
| 7 | (86) | 346.3 | - |
| 8 | (119) | 289.7 | 125.7 |

As shown in Table 1, the benzazole compounds of the present invention were found to be capable of being vapor-deposited even though the compounds have high melting points. The compounds of the present invention were also found to have no glass transition point or have a glass transition point of 100 °C or more. These results indicate that the benzazole compounds of the present invention form stable thin films and exhibit excellent durability.

Using the compounds obtained in Examples 1 to 8 along with Alq₃ and Compound (CPL-1) having the structure shown below (see, for example, Patent Document 2) as comparative compounds, vapor-deposited films having a thickness of 80 nm were formed on a silicon substrate. The refractive index n and the extinction coefficient k at wavelengths of 450 nm and 750 nm of each of the prepared vapor-deposited films were measured using a spectroscopic measurement apparatus (trade name: F10-RT-UV manufactured by Filmetrics,). The measurement results are summarized in Table 2.

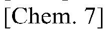

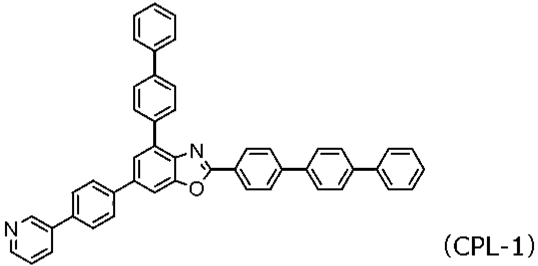

**[Table 2]**

| | Compound | Refractive index n | | Extinction coefficient k | |
|---|---|---|---|---|---|
| | | λ: 450nm | λ: 750nm | λ: 450nm | λ: 750nm |
| Example 1 | (4) | 2.24 | 1.93 | 0.00 | 0.00 |
| Example 2 | (12) | 2.27 | 1.95 | 0.00 | 0.00 |
| Example 3 | (32) | 2.33 | 1.98 | 0.00 | 0.00 |
| Example 4 | (33) | 2.39 | 1.99 | 0.01 | 0.00 |
| Example 5 | (58) | 2.31 | 1.96 | 0.00 | 0.00 |
| Example 6 | (74) | 2.25 | 1.93 | 0.00 | 0.00 |
| Example 7 | (86) | 2.29 | 1.95 | 0.01 | 0.00 |
| Example 8 | (119) | 2.21 | 1.92 | 0.00 | 0.00 |
| Comparative | Alq₃ | 1.88 | 1.73 | 0.03 | 0.00 |
| Comparative | (CPL-1) | 2.13 | 1.86 | 0.00 | 0.00 |

As shown in Table 2, the benzazole compounds of the present invention have refractive indexes and extinction coefficients that are equal to or higher than those of the comparative compounds Alq₃ and Compound (CPL-1) in the wavelength range from 450 nm to 750 nm. In other words, the use of the benzazole compounds of the present invention as constituent materials of a capping layer can be expected to improve light extraction efficiency in an organic EL device.

### Example 9

Hole injection layer 3, hole transport layer 4, light-emitting layer 5, electron transport layer 6, electron injection layer 7, cathode 8, and capping layer 9 were sequentially vapor-deposited on a glass substrate 1 on which a reflective ITO electrode had been formed in advance as transparent anode 2, whereby an organic EL device having the configuration shown in Fig. 10 was fabricated.

Specifically, glass substrate 1 on which an ITO film having a thickness of 50 nm, a reflective film of a silver alloy having a thickness of 100 nm, and an ITO film having a thickness of 5 nm were sequentially formed was subjected to ultrasonic cleaning in isopropyl alcohol for 20 minutes and then dried on a hot plate heated to 250°C for 10 minutes. A UV-ozone treatment was then performed for 2 minutes, and the resulting glass substrate with ITO was installed in a vacuum deposition apparatus and the pressure was reduced to 0.001 Pa or less. Subsequently, an electron acceptor (Acceptor-1) and Compound (HTM-1), each having the structure shown below, were binary-deposited so as to cover transparent anode 2 at a deposition rate ratio of Acceptor-1:(HTM-1) = 3:97, whereby hole injection layer 3 was formed to a thickness of 10 nm. Compound (HTM-1) was vapor-deposited to a thickness of 140 nm on hole injection layer 3 to form a hole transport layer 4. Compound (EMD-1) and Compound (EMH-1), each having the structure shown below, were binary-deposited on hole transport layer 4 at a deposition rate ratio of (EMD-1):(EMH-1) = 5:95, whereby a light-emitting layer 5 having a thickness of 20 nm was formed. Compound (ETM-1) and Compound (ETM-2), each having the structure shown below, were binary-deposited on light-emitting layer 5 at a deposition rate ratio of (ETM-1):(ETM-2) = 50:50, whereby electron transport layer 6 having a thickness of 30 nm was formed. Lithium fluoride was vapor-deposited to a thickness of 1 nm on electron transport layer 6 as electron injection layer 7. A magnesium-silver alloy was vapor-deposited to a thickness of 12 nm on electron injection layer 7 as cathode 8. Finally, Compound (4) obtained in Example 1 was vapor-deposited to a thickness of 60 nm as capping layer 9.

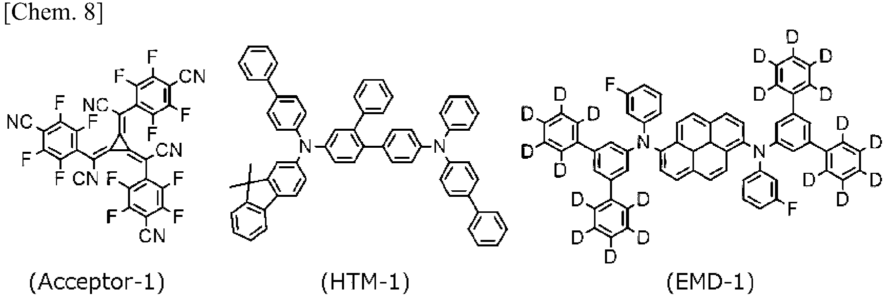

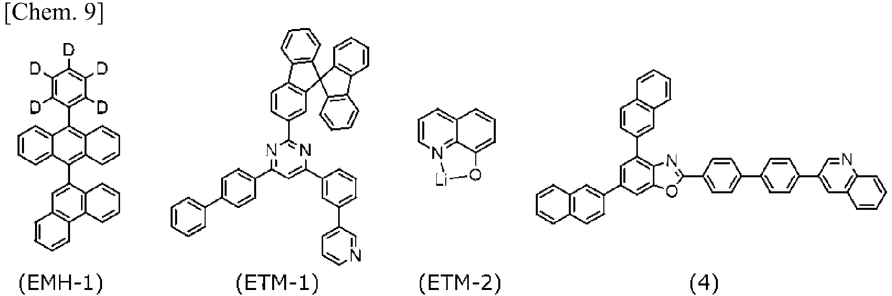

### Examples 10 to 16

Organic EL devices according to Examples 10 to 16 were prepared in the same manner as in Example 9, with the exception that the compounds obtained in Examples 2 to 8 were respectively used instead of Compound (4) as capping layer 9.

### Comparative Example 1

An organic EL device was prepared in the same manner as in Example 9, with the exception that Alq₃ was used instead of Compound (4) as capping layer 9.

### Comparative Example 2

An organic EL device was prepared in the same manner as in Example 9, with the exception that Compound (CPL-1) was used instead of Compound (4) as capping layer 9.

The organic EL devices prepared in the Examples and Comparative Examples were evaluated for light-emitting characteristics by applying a direct-current voltage in air at room temperature (current density: 10 mA/cm²). In addition, the organic EL devices prepared in the Examples and Comparative Examples were used to measure the device lifetimes. The results are summarized in Table 3. In the present invention, device lifetime is defined as the time required for the luminance to decrease to 95% of the initial luminance (100%) when driven at a constant current of 10 mA/cm².

**[Table 3]**

| | Capping layer | Voltage (V) | Luminance (cd/m²) | Luminous efficiency (cd/A) | Power efficiency (lm/W) | Device lifetime (hours) |
|---|---|---|---|---|---|---|
| Example 9 | Compound (4) | 3.64 | 788 | 7.88 | 6.80 | 156 |
| Example 10 | Compound (12) | 3.65 | 792 | 7.92 | 6.82 | 159 |
| Example 11 | Compound (32) | 3.65 | 810 | 8.10 | 6.98 | 156 |
| Example 12 | Compound (33) | 3.66 | 820 | 8.19 | 7.04 | 148 |
| Example 13 | Compound (58) | 3.66 | 807 | 8.07 | 6.92 | 158 |
| Example 14 | Compound (74) | 3.66 | 787 | 7.87 | 6.76 | 151 |
| Example 15 | Compound (86) | 3.65 | 799 | 7.99 | 6.88 | 160 |
| Example 16 | Compound (119) | 3.66 | 780 | 7.80 | 6.71 | 155 |
| Comparative Example 1 | Alq₃ | 3.65 | 714 | 7.14 | 6.15 | 114 |
| Comparative Example 2 | Compound (CPL-1) | 3.66 | 771 | 7.68 | 6.60 | 138 |

As shown in Table 3, the driving voltages at a current density of 10 mA/ cm² were substantially the same for the devices according to Comparative Examples 1 and 2 and the devices according to Examples 9 to 16. On the other hand, with respect to luminance, luminous efficiency, power efficiency, and device lifetime, all devices according to the examples exhibited remarkable improvements compared with the devices according to the comparative examples. These results demonstrate that the benzazole compounds of the present invention are materials suitable for use as a capping layer, and further, that increasing the refractive index of the material used for the capping layer can significantly improve the light extraction efficiency of an organic EL device.

Although preferred embodiments of the present invention have been described above in detail, the present invention is not limited to the above embodiments, and it should be understood that various modifications and variations can be made without departing from the spirit or scope of the invention as set forth in the appended claims.

### INDUSTRIAL APPLICABILITY

The benzazole compounds of the present invention have a high refractive index, can significantly improve light extraction efficiency, and exhibit stable thin-film states, and are therefore suitable as compounds for use in a capping layer of an organic EL device. In addition, organic EL devices fabricated using the benzazole compounds of the present invention can achieve high efficiency. Furthermore, since the benzazole compounds of the present invention do not exhibit absorption in the blue, green, or red wavelength regions, these compounds are particularly suitable for applications requiring display of images with high color purity that are clear and bright. The benzazole compounds of the present invention are expected to be applicable, for example, to household appliances and lighting applications.

### REFERENCE SIGNS LIST

- 1: glass substrate
- 2: anode
- 3: hole injection layer
- 4: hole transport layer
- 5: light-emitting layer
- 6: electron transport layer
- 7: electron injection layer
- 8: cathode
- 9: capping layer

## Claims

1. An organic electroluminescent device, comprising an anode, a hole transport layer, a light-emitting layer, an electron transport layer, a cathode, and a capping layer in that order, wherein the capping layer comprises a benzazole compound represented by the following general formula (1): 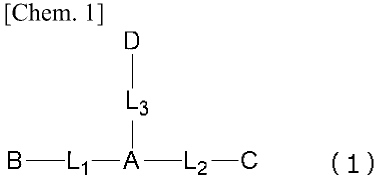
wherein A represents a benzoxazolyl ring or benzothiazolyl ring,
B and C each independently represent a substituted or unsubstituted phenyl group, naphthyl group, quinolyl group, quinoxalinyl group, quinazolinyl group, phenanthrolinyl group, benzoxazolyl group, benzothiazolyl group, benzofuryl group, benzothienyl group, or oxazolopyridyl group,
D represents either a hydrogen atom or a substituted or unsubstituted aryl group or heteroaryl group, and
L₁ to L₃ each independently represent either a single bond or an unsubstituted divalent aryl group or divalent heteroaryl group.

2. The organic electroluminescent device according to claim 1, wherein L₂, L₃, and L₁ in general formula (1) are bonded to the 2-position, 4-position, and 6-position, respectively, of the benzoxazolyl ring or benzothiazolyl ring represented by A.

3. The organic electroluminescent device according to claim 1, wherein L₁ to L₃ in general formula (1) are each independently either a single bond or an unsubstituted phenylene group, biphenylylene group, or naphthylene group.

4. The organic electroluminescent device according to claim 1, wherein the aryl group or heteroaryl group represented by D in general formula (1) is selected from a group consisting of a phenyl group, biphenylyl group, naphthyl group, furyl group, quinolyl group, benzoxazolyl group, benzothiazolyl group, oxazolopyridyl group, quinoxalinyl group, quinazolinyl group, and phenanthrolinyl group.

5. The organic electroluminescent device according to claim 4, wherein D in general formula (1) is a hydrogen atom, a cyanophenyl group, or an unsubstituted phenyl group, biphenylyl group, naphthyl group, furyl group, quinolyl group, benzoxazolyl group, benzothiazolyl group, oxazolopyridyl group, quinoxalinyl group, quinazolinyl group, or phenanthrolinyl group.

6. The organic electroluminescent device according to claim 1, wherein at least one of B and C in general formula (1) is a group selected from a group consisting of an unsubstituted phenyl group, naphthyl group, quinolyl group, quinazolinyl group, phenanthrolinyl group, benzoxazolyl group, benzothiazolyl group, and oxazolopyridyl group.

7. The organic electroluminescent device according to claim 1, wherein when the capping layer is formed to a thickness of 30 nm to 120 nm, the refractive index of the formed film is 1.70 or more in a wavelength range of 450 nm to 750 nm.

8. The organic electroluminescent device according to any one of claims 1 to 7, wherein the capping layer is a laminated layer or a mixed layer comprising two or more kinds of compounds including a benzazole compound represented by general formula (1).

9. A benzazole compound represented by the following general formula (2): 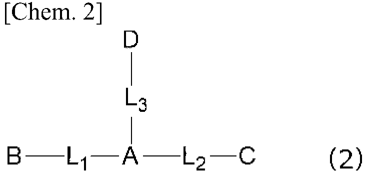
wherein A represents a benzoxazolyl ring or benzothiazolyl ring,
B and C each independently represent a substituted or unsubstituted phenyl group, naphthyl group, quinolyl group, quinoxalinyl group, quinazolinyl group, phenanthrolinyl group, benzoxazolyl group, benzothiazolyl group, benzofuryl group, benzothienyl group, or oxazolopyridyl group,
D represents either a hydrogen atom or a substituted or unsubstituted phenyl group, biphenylyl group, naphthyl group, furyl group, quinolyl group, quinoxalinyl group, quinazolinyl group, phenanthrolinyl group, benzoxazolyl group, benzothiazolyl group, benzofuryl group, benzothienyl group, or oxazolopyridyl group,
L₁ to L₃ each independently represent either a single bond or an unsubstituted phenylene group, biphenylylene group, or naphthylene group, and
L₂, L₃, and L₁ are bonded to the 2-position, 4-position, and 6-position, respectively, of the benzoxazolyl ring or benzothiazolyl ring represented by A,
wherein the compound satisfies either Requirement 1 or 2 below:
Requirement 1: At least one of B, C, and D contains a structure selected from a group consisting of quinazoline, phenanthroline, and oxazolopyridine, provided that:
if one or two of B, C, and D are substituted or unsubstituted phenanthrolinyl groups and none of the one or two of B, C, and D that are not the phenanthrolinyl groups is a group selected from a substituted or unsubstituted quinazolinyl group and oxazolopyridyl group, then at least one of L₁, L₂, and L₃ bonded to the phenanthrolinyl groups is an unsubstituted phenylene group, biphenylylene group, or naphthylene group; and
if B, C, and D are all substituted or unsubstituted phenanthrolinyl groups, at least one selected from L₁, L₂, and L₃ bonded to the phenanthrolinyl groups is an unsubstituted phenylene group, biphenylylene group, or naphthylene group; and
Requirement 2: None of B, C, and D contains any structure selected from a group consisting of quinazoline, phenanthroline, and oxazolopyridine, L₃ is a single bond, and D is a hydrogen atom.

10. The benzazole compound according to claim 9, wherein at least one of B and C in general formula (2) is a group selected from a group consisting of a substituted or unsubstituted phenyl group, naphthyl group, quinolyl group, quinazolinyl group, phenanthrolinyl group, benzoxazolyl group, benzothiazolyl group, benzofuryl group, and oxazolopyridyl group.

11. An electronic apparatus or an electronic device, comprising a pair of electrodes and at least one organic layer, wherein the organic layer comprises a benzazole compound according to claim 9 or 10.
